# EUROPEAN PATENT SPECIFICATION

(11) **EP 1 704 837 B1**
(45) Date of publication and mention of the grant of the patent: **29.10.2008**
(21) Application number: 06447034.7
(22) Date of filing: 09.03.2006
(51) Int. Cl.: A61F 2/44

(54) **Artificial lumbar disc**
Künstliche Bandscheibe für den Lendenwirbelbereich
Disque lombaire artificiel

(30) Priority: 24.03.2005 US 88410
(43) Date of publication of application: 27.09.2006
(73) Proprietor: Neurocare International Inc., New Orleans LA 70116 (US)
(72) Inventor: Leclercq, Toussaint, Huntington, WV 25701 (US)
(74) Representative: Powis de Tenbossche, Roland

(56) References cited:
- EP-A- 1 354 572
- WO-A-92/10982
- WO-A-94/23671
- US-A1- 2005 027 358
- US-B1- 6 419 704

## Description

### BACKGROUND OF THE INVENTION

### 1. Field of the Invention

This invention relates generally an artificial lumbar disc made of three components. The prosthesis is inserted into a disc cavity to restore natural function of the disc.

### 2. Description of the Prior Art

US-A-6419704 discloses artificial replacements for natural intervertebral discs in humans and animals. The artificial replacement comprises a shaped body having a final volume sized to the intervertebral disc space, and a material associated to the shaped body enabling the body to cyclically compress and expand.

Back pain is the leading cause of disability and worker compensation claims in the US for people under 45 years of age. The cost of treating back pain varies according to different studies but numbers as high as $26 billion in direct cost and $90 billion in total cost are frequently cited.

The normal human disc is comprised of an outer fibrous structure (annulus fibrosus) with fibrous bands organized like the plies of a radial tire. The annulus fibrosus controls the motion of the vertebral segment. The inner core or nucleus pulposus has a water content of 70 to 85%. It transmits and dampens axial loading ("shock absorbing" function). In degenerative disc disease, the annulus develops structural lesions that weaken its ability to control segmental movements and to contain the central core. Also, the nucleus loses part of its water content. The combined results provide for segmental instability and a decrease in the shock absorbing function of the disc. Spinal stenosis, osteophyte formation, disc herniation, and possible nerve root compression are associated with disabling back pain.

At the present time, when conservative measures fail to provide relief for the patient, the most common available surgical procedures consist of discectomy (in variable amount of completeness) or arthrodesis (spinal fusion) of the involved segment. Though initially successful in relieving pain in 50 to 90 % of cases, many discectomy patients experience return of pain and may require additional surgery, often in the form of a spinal fusion. Arthrodesis techniques provide for stability of the affected segment and solid fusions are now reported in the 90 to 95% range though pain relief is present in only 75 to 80% of cases depending on the series reported. Unfortunately, as longer follow-up studies become available, accelerated degeneration of the unfused segments above and below the arthrodesis presents an increasing problem.

Spinal surgeons have become progressively more aware of this phenomenon, opening the door for new techniques that can preserve the motion of the vertebral segment with artificial disc prostheses. This has resulted in a more physiological approach to intervertebral disc disease. Artificial disc prostheses are now in different stages of development. At the present time, only one artificial disc has been approved for patient use in the US. It has a ball and socket design, requires a major invasive surgical procedure, and does not address the issue of the "shock absorbing" function of the normal human intervertebral disc. Other prostheses now at different stages of development are also of the ball and socket design and/or require major invasive surgery for adequate implantation.

### SUMMARY OF THE INVENTION

There is a need for newer prosthetic designs that will provide an artificial disc with components more similar to the natural disc, allowing for reestablishment of a more physiologically correct function. There is also a need for designs that allow for less invasive surgery for their insertion thus providing patients with safer alternatives. In addition and importantly, the present state of the art does not provide for a safe, reproducible salvage operation in the case of prosthetic failure. The present invention provides answers to the multiple problems cited above.

Generally, an artificial lumbar disc prosthesis can be formed from three components. Each of the components may be inserted individually. Two bean shaped implants are a mirror image of each other and are designed to be inserted on each side of the disc cavity. The third component is designed to fill the remaining disc cavity after the first two components have been inserted. The unique design focuses on restoring the natural function of the disc, reestablishing a preferred disc space, and providing ease of insertion through both a posterior and anterior approach. Moreover, the potential of prosthesis failure is fully addressed and feasible solutions to such an occurrence are proposed.

### BRIEF DESCRIPTION OF THE INVENTION

The invention relates to an artificial lumbar disc to be inserted into a disc cavity, comprising at least the following components :
a pair of bean shaped implants, each implant comprising a bone contacting surface and an annulus contacting surface and each implant being flexible and having a varying thickness according to the natural contour of the annulus; and
a nucleus comprising a midline inflatable implant which is insertable into the disc cavity to completely fill and lock the said components in place.

The pair of bean shaped implants are adapted to defined a substantially cylindrical passage or chamber with a central axis, in which the midline inflatable implant is intended to be located. The thickness of the bean shaped implants is measured in a direction parallel to said central axis.

Advantageously, a granular porous titanium (for example in the form of a layer) is applied to both surfaces of the bean shaped implants for insuring long term fixation.

According to an embodiment, each component of the artificial lumbar disc has a shape for their sequential insertion in the disc cavity.

Advantageously, the bean shaped implants and the nucleus are adapted for being inserted individually and for being assembled in an intervertebral space.

According to a further detail of an embodiment, the bean shaped implants and the nucleus are adapted for being inserted through a posterior or an anterior approach to the disc cavity.

According to still a further embodiment, the pair of bean shaped implants have a shape for protecting or reestablishing a lordosis of a lumbar spine.

In a preferred embodiment, the pair of bean shaped implants are custom-made to fit unique contours of vertebral end-plate of the patient.

Most preferably, at least one bean shaped implant, preferably the two bean shaped implants are shaped with a height difference or thickness and/or a degree of convexity obtained through radiological and mathematical methods.

According to a further advantageous embodiment, the bean shaped implants and the nucleus or inflatable implant are adapted for being sequentially introduced into the disc space through a minimally invasive procedure.

Advantageously, a desired disc height can be reestablished through spinal dilators which allow for a progressive distracting of the disc cavity.

According to a preferred embodiment, the midline inflatable implant is provided with means adapted for connecting the midline inflatable implant to an inflating means adapted for controlling and/or optimizing the degree of inflation of the midline implant to obtain a physiologically correct implant.

According to a possible embodiment, at least one bean shaped implant, advantageously the two bean shaped implants are shaped so as to be individually removed after their placement. This enables a quick replacement in case one bean shaped implant seems to be the most appropriate or in case of defect.

Advantageously, the bean shaped implants are shaped so that when their free ends are placed the one in front the other for defining therebetween a central chamber and for defining a passage between the first free ends of the first and second bean shaped implants adjacent to each other and/or between the second free ends of the first and second bean shaped implants adjacent to each other, , said passage being adapted for passing a midline inflatable implant, in its at least partial deflated form, through said passage into and/or out the central chamber. At least one space defined between a first end of the first bean shaped implant and a first end of the second bean is adapted for the passage of the inflatable implant, in its deflated or at least partly deflated status, in and out the central chamber defined between the lateral faces of the two bean shaped implants which are facing the one to the other. This enables a easy removal of the midline inflatable implant, for example after a substantially deflating of the midline inflatable implant, so as to fill the central chamber with a bone generating composition or with bone or with bone particles, said composition or particles being advantageously associated or mixed with one or more bone growth agents. The bone generating composition or bone particles are adapted for forming living bone in the chamber, resulting in a solid fusion of the bean shaped implants and the vertebral endplates with the bone generated in the central chamber. When removing the midline inflatable implant, for revision surgery for example, it is possible to replace said midline inflatable implant by a new midline inflatable implant.

According to an advantageous detail, the pair of bean shaped implants include a metal top surface, such as a titanium containing layer, said layer being for example sprayed or painted or applied by dipping the implants in an appropriate bath.

According to another detail, the bean shaped implants or lateral bean shaped implants are reinforced, advantageously with one or more textiles or tissues, preferably woven textile or tissue. According to a specific embodiment, a woven textile band is wrapped at least partly around the bean shaped implant. Advantageously, the textile, preferably the woven textile, is a textile adapted for adhering to the natural tissues and/or adapted for cell colonization. The textile(s) can be provided with specific coating suitable for cell growth. Possibly, before use of the implant, the implant can be treated with a composition, such as a liquid or a gel for improving the adherence to the natural tissue (living) and/or for improving cell colonization.

Preferably, the metal top surface of each bean shaped implant includes at least two fins which are advantageously made in a memory material.

According to a possible embodiment, the bean shaped implant is at least partly provided with a titanium containing layer or covering and with one or more textiles, especially woven textiles.

According to a further advantageous detail of embodiments, at least one portion of the faces (for example the faces intended to face vertebral end plates of a patient) of one or both bean shaped implants is provided with means adapted for adhering to human living tissues and/or adapted for cell colonization, advantageously with a coating suitable for cell growth.

According to still a further embodiment, the artificial lumbar disc of the invention comprises a means for measuring a pressure, especially an intradiscal pressure. Preferably, the midline inflatable implant is provided with a pressure monitor (24).

These and other features and objectives of the present invention will now be described in greater detail with reference to the accompanying drawings, wherein:

### BRIEF DESCRIPTION OF THE DRAWINGS

FIG. 1 is a perspective view of the bean shaped implant;
FIG. 2 is a perspective view of the bean shaped implant;
FIG. 3 is a top view of all three components after insertion.
FIG. 4 is a perspective view of all three components after insertion.
FIG. 5 is a perspective view of all three components after insertion including the fins which ensure attachment.
FIG. 6 is a perspective view of the posterior of the human spine.
FIG. 7 is a perspective view of the dilator used to dilate the disc space in order to obtain an ideal height.
FIGS. 8A-C are perspective views of the different variations of lordosis.
FIG. 9 is a perspective view of the lumbar section of the spine.
FIG. 10 is a perspective view of another bean shaped implant.

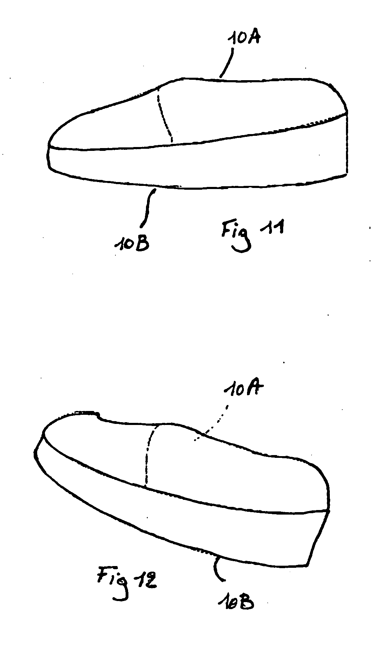

FIG. 11 and 12 are lateral views of bean shaped implant such as shown in Fig. 10.

### DETAILED DESCRIPTION OF THE INVENTION

As used herein, the following definitions apply. In the context of this application, the word anterior is meant to apply to that aspect of the spine facing the abdomen of the patient. The word posterior is meant to apply to that aspect of the spine facing the back of the patient.

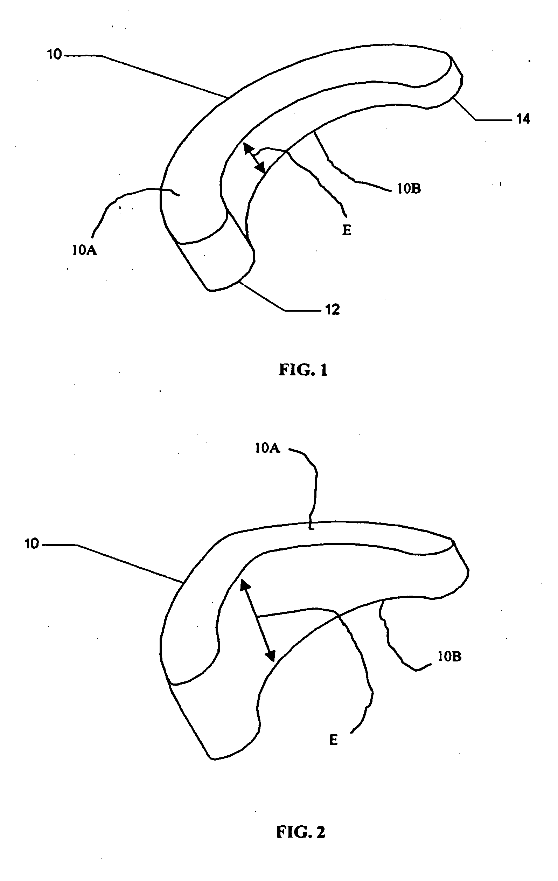

Figure 1 shows a perspective view of a bean shaped implant 10 which illustrates the difference in height between the anterior and posterior sides. As shown the thickness of the anterior side 12 of the implant is larger than the posterior side 14. The convexity of the bean shaped implant 10 as illustrated in Figure 2. These physiological characteristics are key to mimicking the natural juxtaposition and movements of the disc and vertebral endplates. This uniquely allows the implants be built to individual specifications to suit the individual patient. In particular, the vertebral endplates are not parallel in the normal disc space, therefore the prosthesis is designed with the anterior aspect thicker than the posterior aspect. The normal disc space is bi-concave and thus both the inferior and superior surfaces of the bean shaped implants facing the vertebral endplates must be convex. Furthermore, in the present invention, the difference in thickness as well as the convexity between the anterior and posterior part of the bean shaped prosthesis is variable and can be adjusted to individual patient needs. The exact and varying degrees of convexity across the implant are obtained through radiological graphing. The further advantages of the bean shaped implant shall be apparent when we discuss surgical techniques and the biomechanical properties of the implant.

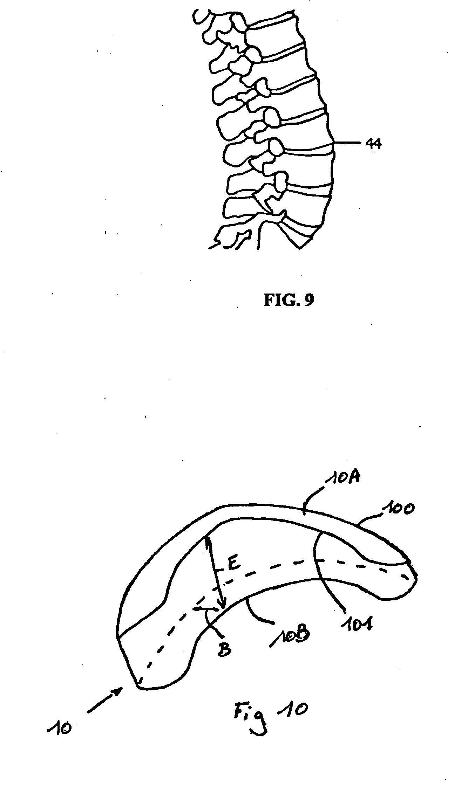

Figure 10 is a bean shaped implant similar to that of figures 1 and 2. The implant (10) comprises a bone contacting surface (10A) and an annulus contacting surface (10B). The implant (10) is flexible and has a varying thickness (E) according to the natural contour of the annulus. The thickness (E) varies also advantageously in the radial direction or in the breath (B) of the implant. Such a variation of height (E) enables to achieve a form suitable for preventing lateral movements of the implant when placed. The bone contacting surface 10A has for example one, preferably more than one apex, most preferably one or more apex lines located between the edges 100, 101 of the surface 10A.. Advantageously the implant has at least two, preferably more than 3 (such as 4,5 or even more) distinct apex or apex lines, which are distant the one from the other.

The bean shaped implant is for example made of osteo acceptable material, such as osteo acceptable resin materials, osteo acceptable polymer materials, osteo acceptable cured materials, hydroxyapatite, etc., and any other materials which are osteo acceptable. Preferably, the material is selected so as to be at least partly osteo integrable or suitable for cell colonisation. The material is advantageously porous or at least partly porous, or possibly only porous or partly porous along the surface.

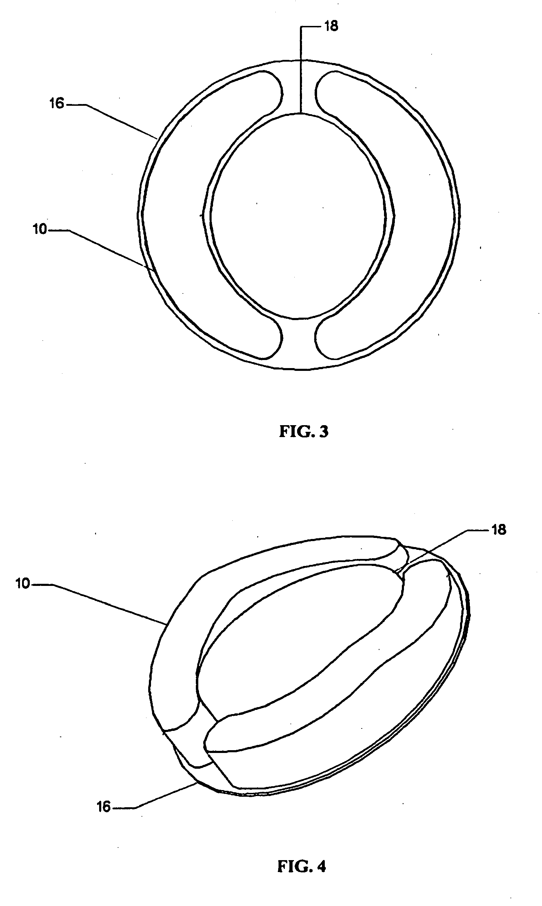

Figure 3 is a top view and Figure 4 is a perspective view showing all three components after insertion. One of the bean shaped implants 10 is meant to be inserted on the left side and the second is inserted on the right side. Because of the design, the prosthesis can be inserted through a posterior as well as an anterior approach using techniques that are familiar to spine surgeons. The part of the annulus fibrosus 16 which is left intact supports the containment of the prosthesis and lessens the chances of migration.

The central inflatable nucleus 18 of the artificial disc is to occupy the disc space left empty between the two afore mentioned implants 10. The size of the midline implant can be chosen using pre-operative measurements to fill adequately the existing cavity. When completely inflated, the implant shall fill the cavity completely, maximizing areas of contact between the prosthesis and the end plates thus minimizing the chance of subsidence or migration.

The midline implant 18 consists of a membrane, which can be inserted on top of an inserting tool. Once in place, the midline implant can be filled under pressure with liquid gel, gas, polymer, or any other biocompatible material. The pressure inside the implant can be regulated and optimized. The central implant 18 has a detachable catheter, that is preferably attached at the time of insertion. Once the implant is in place, the catheter used to inflate the device can be removed, leaving the (one-way valve) implant in place. The inflatable implant, when totally expanded, locks in place the other components of the prosthesis, eliminating or at least significantly reducing the chances of migration.

The body of the prosthesis is made of a silastic material or any other biocompatible material of adequate consistency, resiliency, flexibility, and compressibility. These characteristics are necessary to provide adequate constrain in flexion - extension, lateral tilt, rotation, and axial loading, thus mimicking the natural intervertebral disc.

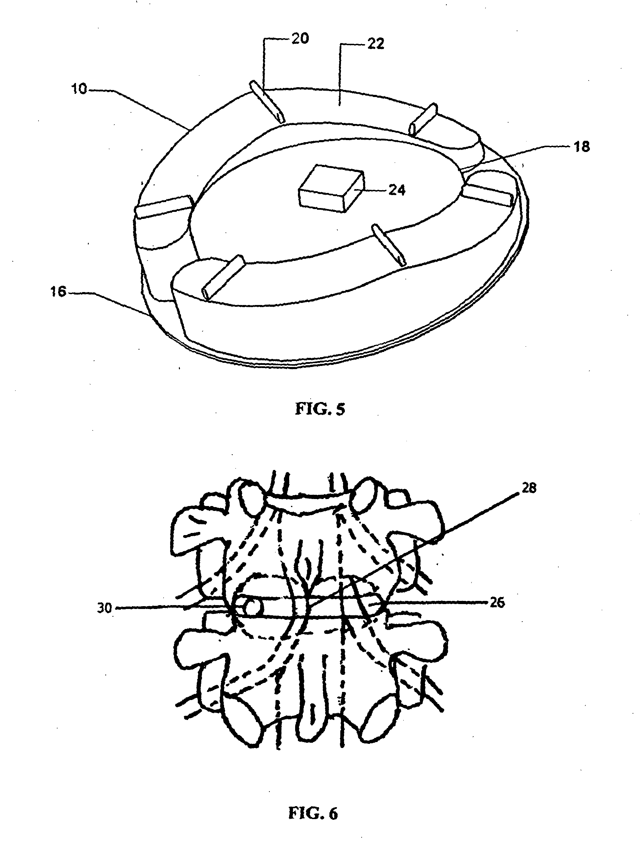

Figure 5 illustrates the complete prosthesis including metal fins 20 which ensure satisfactory attachment to the vertebral endplates and prevents migration. The fins 20 are part of a metal plate 22 which is the upper portion of the bean shaped implant 10. The plates 22 and fins 20 are made of medical grade, porous titanium alloy or any other metal, porcelain, or other synthetic material. The metal plates have at least two fins facing the endplate to prevent displacement. Alternatively porous titanium may be sprayed on the surface of the implant 10 or metal having "memory" may be used. When using a metal with a memory, the fins on the plate are reduced such that they are smooth and "flat" on the metal surface. Once the implant including the metal is placed in the body and the temperature of the same increases, the fins "grow" and "develop" on the plate to resume their previous shape.

In another embodiment, a pressure monitor 24 may be inserted in the midline implant to allow for post-operative intradiscal pressure measurement.

In yet another embodiment of this invention, the surfaces of the bean shaped prostheses facing the vertebral endplates are coated with porous titanium.

In still another embodiment of this invention, the bean shaped implants or lateral bean shaped implants are reinforced, advantageously with one or more textiles, preferably woven textile. According to a specific embodiment, a woven textile band is wrapped at least partly around the bean shaped implant. Advantageously, the textile, preferably the woven textile, is a textile adapted for adhering to the natural tissues and/or adapted for cell colonization.

In yet another embodiment of this invention, the surfaces of the midline implant facing the vertebral endplates are coated with porous titanium.

In yet another embodiment of this invention, the inferior and superior surfaces of the bean-shaped implant are made of memory titanium alloy allowing for full expansion of the fins after implantation.

### SURGICAL TECHNIQUE

The surgical techniques used for insertion of the prosthetic disc are similar to the techniques used universally by spine surgeons to insert paired implants for the purpose of fusing the spine, which include both:
(ALIF - Anterior lumbar interbody fusion)
(PLIF - Posterior lumbar interbody fusion).

Because these procedures are familiar to spine surgeons, their description shall not be repeated except to describe a few modifications that have been designed to accommodate the insertion of this artificial disc along with specially designed instruments to make the technique user friendly.

Figure 6 shows the posterior of the human spine where the surgical procedure is performed. Once the intervertebral disc 26 has been approached, a small incision is made on one side of the disc and then the other. The nervous structures 28 can be safely retracted as long as the two sides are approached one after another. The nervous structures should not be retracted over the midline. The disc material is then removed thoroughly through the two openings 30 using accepted techniques.

In degenerative disc disease, the disc height is usually reduced when compared with normal values. Using an algorithm that we have developed the ideal disc space height for the level involved is established. By using dilators specially designed for this purpose and inserting them in a sequential fashion from left to right, the disc space can be reestablished to its normal pre-diseased values before inserting the artificial disc.

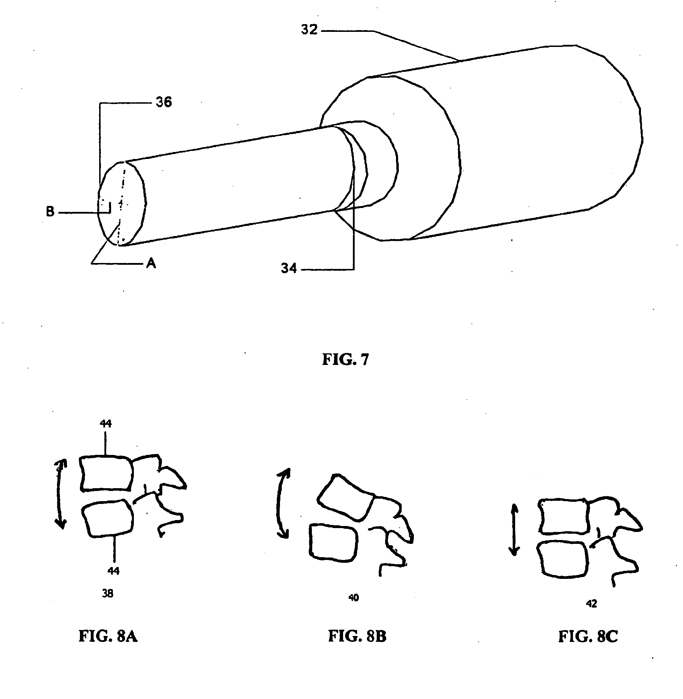

Figure 7 shows a specially designed dilator used to reestablish the disc space. It consists of a handle 32, release mechanism 34, and detachable tip 36. The detachable tip 36 has an elliptical shape with large diameter A and small diameter B. The large diameter A and small diameter B differ by 1 mm. A quarter turn of the dilator provides an increase of 1 mm in the disc space height. A set of dilators allows for the progressive increase of the disc height until satisfactory pre-disease values are established.

The two bean shaped lateral prostheses 10 are then inserted and the midline prosthesis 18 inflated to a satisfactory pressure, locking the components of the prosthesis in place. The closure may then proceed.

The surgical techniques described above are only meant to provide an example of a technique that can be used to insert this artificial disc. Other techniques that provide for a safe and adequate exposure of the intervertebral disc may be used including percutaneous approaches or endoscopic surgery.

Due to its design, the present invention allows for flexion, extension, lateral tilt, rotation, and also allows for axial loading (shock absorber function), contrary to the ball and socket designs presently available.

Because the invention has two bean shaped implants 10 of various heights, it allows for reestablishment or preservation of the lumbar lordosis contrary to existing devices that have parallel surfaces facing the endplates. In the normal human disc, the vertebral endplates are not parallel resulting in the fact that the anterior aspect of the disc is higher than the posterior aspect. Figures 8A-C illustrates the amount of lordosis which uniquely varies among individual patients. These amounts are normal lordosis 38, extreme lordosis 40, and the loss of lordosis 42 which results in "flat back" syndrome. As illustrated by the normal lordosis 38 in Figure 8 and also by Figure 9, the lumbar section of the spine has a natural curvature. This curvature is the reason why the verterbral endplates 44 are not parallel. Existing artificial discs have anterior and posterior heights which are the same, not taking into consideration the normal anatomy of the human disc. This prosthesis addresses this issue.

The loss of lordosis results in back pain and junctional degeneration (this is precisely what the artificial disc is supposed to prevent). Therefore, the prosthesis and the surgical technique are designed in a manner in which to duplicate the pre-disease lordotic conditions. The importance of maintaining lordosis and the negative effects resulting in its loss is addressed in the following articles. 90% of patients with flat back complain of back pain. Lack of lordosis results in detrimental stresses on the adjacent vertebral segment accelerating degenerative changes and creating back pain. Decrease in intradiscal pressure has been associated with hypolordotic conditions. Spinal alignment should be preserved or restored, especially sagittal lordosis.

Because of the design of this artificial disc, the ease of insertion allows for a posterior surgical approach to the lumbar spine contrary to existing prosthesis which require an anterior approach. The anterior approach is plagued with a number of iatrogenic complications including retrograde ejaculation in males, severe bleeding requiring extensive transfusions, vessel injury, thrombosis with possible embolization, long term venous insufficiency, and urethral injury. Deaths have been reported due to such complications. These iatrogenic problems have concerned surgeons to the extent that it limits the indications for surgery. Low back pain is not a fatal condition and the surgeons have to consider the risk-benefit ratio of the anterior approach.

It should be emphasized that the design of this invention allows for anterior as well as posterior insertion. Another advantage of the design is that the surgical techniques used (allowing for some modifications) are familiar to spine surgeons, avoiding the "learning curve" inherent to new instrumentation.

Because the incisions in the annulus are minimal, a significant amount of the annulus is left intact, providing containment of the artificial disc. This will eliminate, or at least significantly decrease the risk of migration of the prosthesis. The anterior surgical approach used for insertion of the presently available total disc prostheses makes preservation of the anterior annulus and the anterior longitudinal ligament impossible or very unlikely.

Advantageously, with this design, the unique combination of two lateral bean shaped implants with an inflatable center results in a complete filling of the cavity left by the disc removal. This tight fit of the implants within the disc space argues favorably for a stable construct with little or no likelihood of migration.

A further advantage of this invention is that the prosthesis can be "custom made" allowing for variable anterior and posterior thickness as well as for varying degrees of convexity thus allowing for a more physiologically correct outcome. A person's disc area is modeled and then the prosthesis is formed to fit that individual's disc area.

The issue of prosthesis failure is commonly left unaddressed even though these prosthesis are destined to remain in place for many years and even decades. None of the prosthetic designs of total disc prosthesis have known specific considerations for replacement or revision.

With this prosthesis design, a failure of the artificial disc to function can be addressed by deflating and removing the central prosthesis, then filling the cavity with bone to obtain an interbody lumbar fusion, an operation that is well accepted and has a satisfactory historical track record. This issue once again highlights the benefits of a posterior approach since the following risks associated with the reopening of an anterior approach are greater. Reopening of an anterior, transabdominal approach carries significant risk of severe complications with possible intractable bleeding due to adhesions to the major blood vessels and is a life threatening operation.

Though this invention is designed to be applied to the treatment of the human lumbar spine, it may be applied to other vertebrates of the animal kingdom.

In light of the foregoing, it will now be appreciated by those skilled in the art that various changes may be made to the embodiment herein chosen for purposes of disclosure without departing from the inventive concept defined by the appended claims. Non-limiting examples of such changes including using an endoscopic procedure in a variant of the technique, varying the number of fins on the implants, or using polymers, nitrous oxide or a hydro gel to inflate the central implant.

A method for designing and preparing the implants for a patient is advantageously adapted for preparing specifically designed implants for one patient.

In said method, the patient is viewed by means of a scanner and a NMR (nuclear magnetic resonance) device. A series of views or images is obtained and treated to define a 3D image.

From said 3D image(s), the shaped and design of the patient's discs are defined, whereby the shape and design of the prothesis to be inserted between two patient's discs is defined.

The two bean shaped lateral implants for making said artificial disc are defined. The implants are advantageously defined by one or more data files suitable to be used in a computer for shaping or forming mold(s) and/or for processing a basic implant.

The bean shaped lateral implants are manufactured. For example by making specific mold(s), in which a material is placed or injected. The lateral implants can also be manufactured by mechanical processing, such as mechanical grinding, said processing and/or grinding being advantageously controlled by computer. According to a preferred embodiment, the lateral implants are first made by filling a material in one or more molds, then the molded implants is corrected by computer mechanical processing.

The form of the inflatable implant is also preferably specific for the patient.

Said inflatable implant is for example made of a polymer outer skin, which is adapted to contain a pasta or a gel. The inflatable implant is like an inflatable envelope. The pasta or gel is advantageously selected so as to form after curing, reaction, etc. a system blocking in place the lateral implants. The polymer which is bio compatible is advantageously quite rigid, while being however deformable by inflating the inflatable implant.

Faces of the implant can be provided with a coating, such as a titanium coating, and/or with a woven tissue and/or with fins. By using such implants, the treatment is individualized.
Such an artificial lumbar disc is made of three components which are inserted individually between two facing vertebral end plates of the patient. Two bean shaped implants are substantially a mirror image of each other and are designed to be inserted on each side of the disc cavity. The third component is designed to fill the disc cavity remaining after the first two components (bean shaped implants) have been inserted. The unique design focuses on restoring the natural function of the disc, reestablishing a preferred disc space, and provides ease of insertion through both a posterior and anterior approach. Moreover, the potential of prosthesis failure is fully addressed and feasible solutions to such an occurrence are proposed.

## Claims

1. An artificial lumbar disc to be inserted into a disc cavity, comprising at least the following components:
a pair of bean shaped implants (10), each implant (10) comprising a bone contacting surface (10A) and an annulus contacting surface (10B) and each implant (10) being flexible and having a varying thickness (E) according to the natural contour of the annulus; and
a nucleus comprising a midline inflatable implant (18) which is insertable into the disc cavity to completely fill and lock the said components (10,18) in place.

2. The artificial lumbar disc of claim 1, wherein a granular porous titanium is applied to both surfaces (10A,10B) of the bean shaped implants (10) for insuring long term fixation.

3. The artificial lumbar disc of any one of the claims 1 to 2, wherein the pair of bean shaped implants are custom-made to fit unique contours of vertebral end-plate of the patient.

4. The artificial lumbar disc of any one of the claims 1 to 3, wherein at least one bean shaped implant, preferably the two bean shaped implants are shaped with a height difference or thickness and/or a degree of convexity obtained through radiological and mathematical methods.

5. The artificial lumbar disc of any one of the claims 1 to 4, wherein the midline inflatable implant is provided with means adapted for connecting the midline inflatable implant to an inflating means adapted for controlling and/or optimizing the degree of inflation of the midline implant to obtain a physiologically correct implant.

6. The artificial lumbar disc of any one of the claims 1 to 5, wherein each of the first and second bean shaped implants has a first free end, a second free end, and an inner lateral face, wherein the two implants are adapted when their respective inner lateral faces are placed in front the one to the other for defining therebetween a central chamber and for defining a passage between the first free ends of the first and second bean shaped implants adjacent to each other and/or between the second free ends of the first and second bean shaped implants adjacent to each other, , said passage being adapted for passing a midline inflatable implant, in its at least partial deflated form, through said passage into and/or out the central chamber.

7. The artificial lumbar disc of any one of the claims 1 to 6, wherein at least one, advantageously the pair of bean shaped implants include a metal top surface.

8. The artificial lumbar disc of claim 7, wherein the metal top surface (22) of each bean shaped implant (10) includes at least two fins (20) which are advantageously made in a memory material.

9. The artificial lumbar disc of any one of the claims 1 to 8, which comprises a means for measuring an intradiscal pressure (24).

10. The artificial lumbar disc of claim 9, wherein the midline inflatable implant (18) is provided with a pressure monitor (24).

11. The artificial lumbar disc of any one of the claims 1 to 10, wherein at least one, advantageously the two bean shaped implants are reinforced, advantageously with one or more textiles or tissues, preferably woven textile or tissue, whereby a woven textile/tissue band is advantageously wrapped at least partly around the bean shaped implant.

12. The artificial lumbar disc of any one of the claims 1 to 11, wherein at least one portion of the faces of one or both bean shaped implants is provided with means adapted for adhering to human living tissues and/or adapted for cell colonization, advantageously with a coating suitable for cell growth.

13. The artificial lumbar disk of any one of the claims 1 to 12, in which the inflatable implant is adapted for being inflated by means of polymers or nitric oxide or a hydro gel.

14. The artificial lumbar disc of any one of the claims 1 to 12, in which the inflatable implant is made of a polymer outer skin, which is adapted to contain a pasta or a gel.

15. The artificial lumbar disc of claim 14, in which the pasta or gel is adapted to form after curing or reaction a system blocking in place the implants (10).

16. The artificial lumbar disc of any one of the claims 1 to 12, in which the inflatable implant consists of a membrane adapted to be filled under pressure with liquid gel, gas, polymer or any other biocompatible material.

17. The artificial lumbar disc of any one of the claims 1 to 16, in which the inflatable implant has a detachable catheter for inflating the inflatable implant, and an one way valve left in place when the removable catheter is removed.

## Patentansprüche

1. Künstliche Bandscheibe für den Lendenbereich, die in einen Hohlraum für die Scheibe eingefügt werden soll, die wenigstens die folgenden Komponenten aufweist:
ein Paar von bohnenförmigen Implantaten (10), wobei jedes Implantat (10) eine einen Knochen berührende Oberfläche (10A) und eine einen ringförmigen Raum kontaktierende Oberfläche (10B) aufweist und jedes Implantat (10) flexibel ist und eine gemäß der natürlichen Kontur des ringförmigen Raums variierende Dicke (E) hat; und
einen Kern, der ein aufblasbares Mittellinienimplantat (18) aufweist, das in den Scheibenhohlraum einfügbar ist, um die Komponenten (10, 18) vollständig auszufüllen und sie an ihrem Ort festzulegen.

2. Künstliche Bandscheibe für den Lendenbereich nach Anspruch 1, bei der ein körniges poröses Titan auf beide Oberflächen (10A, 10B) der bohnenförmigen Implantate (10) aufgebracht ist, um eine Fixierung für lange Zeit sicherzustellen.

3. Künstliche Bandscheibe für den Lendenbereich nach einem der Ansprüche 1 oder 2, bei der das Paar von bohnenförmigen Implantaten nach Kundenspezifikationen hergestellt ist, um mit den einzigartigen Konturen der vertebralen Endplatte des Patienten zusammenzupassen.

4. Künstliche Bandscheibe für den Lendenbereich nach einem der Ansprüche 1 bis 3, bei der wenigstens ein bohnenförmiges Implantat, vorzugsweise zwei bohnenförmige Implantate mit einem Höhenunterschied oder einer Dicke und/oder einem Ausmaß von Konvexität geformt sind, die durch radiologische und mathematische Verfahren erhalten werden.

5. Künstliche Bandscheibe für den Lendenbereich nach einem der Ansprüche 1 bis 4, bei der das aufblasbare Mittellinienimplantat mit Mitteln versehen ist, die dazu ausgebildet sind, das aufblasbare Mittellinienimplantat mit einem Aufblasmittel zu verbinden, das dazu ausgebildet ist, das Ausmaß des Aufblasens des Mittellinienimplantats zu steuern und/oder zu optimieren, um ein physiologisch korrektes Implantat zu erhalten.

6. Künstliche Bandscheibe für den Lendenbereich nach einem der Ansprüche 1 bis 5, bei der jedes der ersten und zweiten bohnenförmigen Implantate ein erstes freies Ende, ein zweites freies Ende und eine innere laterale Fläche hat, wobei die beiden Implantate dazu ausgebildet sind, wenn ihre inneren lateralen Flächen vor dem einen zum anderen angeordnet werden, dazwischen eine mittige Kammer zu bilden und einen Durchlass zwischen den ersten Enden der ersten und zweiten bohnenförmigen Implantate, die einander benachbart sind, und/oder zwischen den zweiten freien Enden der ersten und zweiten bohnenförmigen Implantate, die einander benachbart sind, zu bilden, welcher Durchlass dazu ausgebildet ist, ein aufblasbares Mittellinienimplantat, bei dem wenigstens teilweise das Fluid abgelassen ist, durch den Durchlass in und/oder aus der mittigen Kammer durchzulassen.

7. Künstliche Bandscheibe für den Lendenbereich nach einem der Ansprüche 1 bis 6, bei der wenigstens eines, vorzugsweise das Paar von bohnenförmigen Implantaten eine metallische obere Oberfläche einschließt.

8. Künstliche Bandscheibe für den Lendenbereich nach Anspruch 7, bei der die metallische obere Oberfläche (22) jedes bohnenförmigen Implantats (10) wenigstens zwei Flossen (20) einschließt, die vorzugsweise aus einem Memory-Material hergestellt sind.

9. Künstliche Bandscheibe für den Lendenbereich nach einem der Ansprüche 1 bis 8, die Mittel zum Messen des Drucks (24) innerhalb einer Scheibe aufweisen.

10. Künstliche Bandscheibe für den Lendenbereich nach Anspruch 9, bei der das aufblasbare Mittellinieimplantat (18) mit einer Drucküberwachungseinrichtung (24) versehen ist.

11. Künstliche Bandscheibe für den Lendenbereich nach einem der Ansprüche 1 bis 10, bei der wenigstens eines, vorzugsweise die beiden bohnenförmigen Implantate verstärkt sind, vorteilhafterweise mit einem oder mehreren Textilien oder Tuchelementen, vorzugsweise gewebtem Textil oder Tuchelement, wobei ein gewebtes Textil/Tuchelementband vorzugsweise wenigstens teilweise um das bohnenförmige Implantat gewickelt ist.

12. Künstliche Bandscheibe für den Lendenbereich nach einem der Ansprüche 1 bis 11, bei der wenigstens ein Teil der Fläche eines oder beider bohnenförmigen Implantate mit Mitteln versehen ist, die dazu ausgebildet sind, an menschlichem lebenden Gewebe anzuhaften und/oder für Zellkolonisation ausgebildet sind, vorzugsweise mit einer Beschichtung, die für Zellwachstum geeignet ist.

13. Künstliche Bandscheibe für den Lendenbereich nach einem der Ansprüche 1 bis 12, bei der das aufblasbare Implantat dazu ausgebildet ist, mit Hilfe von Polymeren oder Stickoxid oder einem Hydrogel aufgeblasen zu werden.

14. Künstliche Bandscheibe für den Lendenbereich nach einem der Ansprüche 1 bis 12, bei der das aufblasbare Implantat aus einer äußeren Polymerhaut gebildet ist, die dazu ausgebildet ist, eine Paste oder ein Gel zu enthalten.

15. Künstliche Bandscheibe für den Lendenbereich nach Anspruch 14, bei der die Paste oder das Gel dazu ausgebildet ist, nach Aushärten oder Reaktion ein System zu bilden, das die Implantate (10) an ihrem Ort blockiert.

16. Künstliche Bandscheibe für den Lendenbereich nach einem der Ansprüche 1 bis 12, bei der das aufblasbare Implantat aus einer Membran besteht, die dazu ausgebildet ist, unter Druck mit flüssigem Gel, Gas, Polymer oder irgendeinem anderen biokompatiblen Material gefüllt zu werden.

17. Künstliche Bandscheibe für den Lendenbereich nach einem der Ansprüche 1 bis 16, bei der das aufblasbare Implantat einen entfernbaren Katheter zum Aufblasen des aufblasbaren Implantats und ein Einwegventil hat, das an seinem Ort verbleibt, wenn der entfernbare Katheter entfernt wird.

## Revendications

1. Disque lombaire artificiel à insérer dans une cavité de disque, comprenant au moins les composants suivants
une paire d'implants en forme de haricots (10), chaque implant (10) comprenant une surface (10A) de contact avec l'os et une surface (10B) de contact annulaire et chaque implant (10) étant flexible et ayant une épaisseur (E) variable selon le contour naturel de l'anneau; et
un noyau comprenant un implant (18) gonflable en son milieu qui peut être inséré dans la cavité de disque pour remplir et bloquer complètement lesdits composants (10, 18) en place.

2. Disque lombaire artificiel suivant la revendication 1, dans lequel du titane poreux granulaire est appliqué sur les deux surfaces (10A, 108) des implants en forme de haricots (10) pour assurer une fixation à long terme.

3. Disque lombaire artificiel suivant l'une quelconque des revendications 1 ou 2, dans lequel la paire d'implants en forme de haricots est faite sur mesure pour s'adapter aux contours uniques du plateau vertébral du patient.

4. Disque lombaire artificiel suivant l'une quelconque des revendications 1 à 3, dans lequel au moins un implant en forme de haricot, de préférence les deux implants en forme de haricots sont formés avec une différence de hauteur ou d'épaisseur et/ou un degré de convexité obtenus par des procédés radiologiques et mathématiques.

5. Disque lombaire artificiel suivant l'une quelconque des revendications 1 à 4, dans lequel l'implant gonflable en son milieu est pourvu de moyens adaptés pour connecter l'implant gonflable en son milieu à un moyen de gonflage conçu pour contrôler et/ou optimiser le degré de gonflage de l'implant gonflable en son milieu afin d'obtenir un implant physiologiquement correct.

6. Disque lombaire artificiel suivant l'une quelconque des revendications 1 à 5, dans lequel chacun des premier et deuxième implants en forme de haricots a une première extrémité libre, une deuxième extrémité libre et une face latérale intérieure, dans lequel les deux implants sont adaptés lorsque leurs faces latérales intérieures respectives sont placées en face l'une de l'autre pour définir entre elles une chambre centrale et pour définir un passage entre les premières extrémités libres adjacentes des premier et deuxième implants en forme de haricots et/ou entre les deuxièmes extrémités libres adjacentes des premier et deuxième implants en forme de haricots, ledit passage étant adapté pour faire passer un implant gonflable en son milieu, dans sa forme au moins partiellement dégonflée, à travers ledit passage dans et/ou hors de la chambre centrale.

7. Disque lombaire artificiel suivant l'une quelconque des revendications 1 à 6, dans lequel au moins un implant, de manière avantageuse la paire d'implants en forme de haricots comprend une surface supérieure métallique.

8. Disque lombaire artificiel suivant la revendication 7, dans lequel la surface supérieure métallique (22) de chaque implant en forme de haricot (10) comprend au moins deux ailettes (20) qui sont de manière avantageuse fabriquées dans un matériau à mémoire de forme.

9. Disque lombaire artificiel suivant l'une quelconque des revendications 1 à 8, comprenant un moyen de mesurer une pression intradiscale (24).

10. Disque lombaire artificiel suivant la revendication 9, dans lequel l'implant gonflable en son milieu (18) est pourvu d'un détecteur de pression (24).

11. Disque lombaire artificiel suivant l'une quelconque des revendications 1 à 10, dans lequel au moins un implant, de manière avantageuse les deux implants en forme de haricots sont renforcés, de manière avantageuse avec un ou plusieurs textiles ou tissus, de préférence un textile ou tissu tissé, une bande de textile/tissu tissé entourant de manière avantageuse au moins partiellement l'implant en forme de haricot.

12. Disque lombaire artificiel suivant l'une quelconque des revendications 1 à 11, dans lequel au moins une partie des faces d'un des implants en forme de haricots ou des deux est pourvue de moyens conçus pour adhérer aux tissus humains vivants et/ou conçus pour la colonisation par des cellules, de manière avantageuse avec un revêtement approprié pour la croissance de cellules.

13. Disque lombaire artificiel suivant l'une quelconque des revendications 1 à 12, dans lequel l'implant gonflable est conçu pour être gonflé au moyen de polymères ou de monoxyde d'azote ou d'un hydrogel.

14. Disque lombaire artificiel suivant l'une quelconque des revendications 1 à 12, dans lequel l'implant gonflable est constitué d'une enveloppe extérieure de polymère, qui est conçue pour contenir une pâte ou un gel.

15. Disque lombaire artificiel suivant la revendication 14, dans lequel la pâte ou le gel est conçu pour former après durcissement ou réaction un système bloquant les implants (10) en place.

16. Disque lombaire artificiel suivant l'une quelconque des revendications 1 à 12, dans lequel l'implant gonflable est constitué d'une membrane conçue pour être remplie sous pression avec un gel liquide, un gaz, un polymère ou tout autre matériau biocompatible .

17. Disque lombaire artificiel suivant l'une quelconque des revendications 1 à 16, dans lequel l'implant gonflable a un cathéter détachable pour gonfler l'implant gonflable et une soupape unidirectionnelle laissée en place lorsque le cathéter amovible est enlevé.
